(19) Europäisches Patentamt
European Patent Office
Office européen des brevets

(11) Veröffentlichungsnummer: **0 609 736 A1**

# (12) EUROPÄISCHE PATENTANMELDUNG

(21) Anmeldenummer: **94100940.9**

(51) Int. Cl.5: **C07C 253/30**, C07C 255/24

(22) Anmeldetag: **24.01.94**

(30) Priorität: **04.02.93 DE 4303131**

(43) Veröffentlichungstag der Anmeldung: **10.08.94 Patentblatt 94/32**

(84) Benannte Vertragsstaaten: **DE FR GB IT**

(71) Anmelder: **BAYER AG**

**D-51368 Leverkusen(DE)**

(72) Erfinder: **Blazejak, Manfred, Dr.**
**Richardstrasse 81**
**D-40231 Düsseldorf(DE)**
Erfinder: **Köhler, Wilfried, Dr.**
**Welscher Heide 22**
**D-51429 Bergisch Gladbach(DE)**
Erfinder: **Wemmje, Karl-Johann**
**Industriestrasse 42**
**D-51399 Burscheid(DE)**

## (54) Verfahren zur Herstellung von Mono-N-Beta-cyanoethylanilinen.

(57) Ein neues Verfahren zur Herstellung von Mono-N-β-cyanoethylanilinen, dadurch gekennzeichnet, daß man eine gegebenenfalls substituierte Anilinverbindung mit Acrylnitril in Gegenwart von aktiver Bleicherde als Katalysator bei einer Temperatur zwischen 80 und 200 °C in einem nichtwäßrigen Reaktionsmedium umsetzt.

EP 0 609 736 A1

Die Erfindung betrifft ein Verfahren zur Herstellung von Mono-N-β-cyanoethylanilinen.

Mono-N-β-cyanoethylaniline sind bedeutende Zwischenprodukte zur Herstellung von Farbstoffen, Pharmazeutika und Pflanzenschutzmittel. Obwohl Aniline nicht leicht mit Acrylnitril reagieren, gibt es bereits einige Herstellungsverfahren für Mono-N-β-cyanoethylaniline. Diese besitzen allerdings gewisse Nachteile. So sind beispielsweise in US-A-3 496 213 Zinkchlorid und in A. Heininger J. org. Chem. 22 (1957) 1213 Kupferacetat als Katalysatoren bei der Umsetzung von Anilinen mit Acrylnitril beschrieben worden, die jedoch metallhaltige Abwässer erzeugen.

Auch Eisessig ist als Katalysator bei der Cyanoethylierung von Anilinen aus US-A-2 492 972 bekannt. Dieser Katalysator erweist sich aber als nachteilig bei der Abtrennung aus dem Reaktionsgemisch.

Die genannten Verfahren haben daher den Nachteil, daß sie einerseits Entsorgungsprobleme mitsich bringen und/oder umständlich zu handhaben sind und korrosionsfeste Werkstoffe benötigen.

Gegenstand der Erfindung ist ein neues Verfahren zur Herstellung von Mono-N-β-cyanoethylanilinen, dadurch gekennzeichnet, daß man eine gegebenenfalls substituierte Anilinverbindung mit Acrylnitril in Gegenwart von aktiver Bleicherde als Katalysator bei einer Temperatur zwischen 80 und 200 °C in einem nichtwäßrigen Reaktionsmedium umsetzt. Unter Anilinverbindungen werden dabei primäre oder sekundäre Aniline verstanden, die gegebenenfalls einfach oder mehrfach kernsubstituiert sind.

In einer bevorzugten Ausführungsform kommen Aniline der Formel (I) zum Einsatz

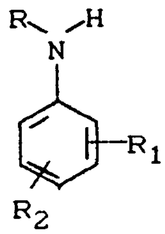

(I),

worin

R = Wasserstoff, gegebenenfalls mit Hydroxy substituiertes $C_1$-$C_{10}$-Alkyl,

$R_1$, $R_2$ = unabhängig voneinander H, $C_1$-$C_{10}$-Alkyl oder $C_1$-$C_{10}$-Alkoxy.

In einer besonders bevorzugten Ausführungsform kommen Aniline der Formel (I) zum Einsatz, in denen

R = Wasserstoff, Methyl, Ethyl, n-Propyl, Isopropyl, β-Hydroxyethyl, β-Hydroxypropyl oder γ-Hydroxypropyl,

$R_1$, $R_2$ = unabhängig voneinander Wasserstoff, Methyl, Ethyl, Isopropyl, n-Propyl, Methoxy oder Ethoxy bedeuten.

In einer ganz besonders bevorzugten Ausführungsform kommen Aniline der Formel (I) zum Einsatz, in denen

R = Wasserstoff oder Methyl, insbesondere Methyl,

$R_1$ = Wasserstoff, Methyl, Methoxy oder Ethoxy und

$R_2$ = Wasserstoff bedeuten.

Unter aktiver Bleicherde werden im allgemeinen Montmorillonit enthaltende Bleicherden wie beispielsweise Bentonit verstanden, die durch Behandlung mit anorganischen Säuren wie beispielsweise Schwefelsäure oder Salzsäure aktiviert worden sind. Montmorillonite sind beispielsweise in Römpp Chemie Lexikon 9. Auflage, S. 2850, Georg Thieme Verlag, Stuttgart 1991 beschrieben. Besonders bevorzugt sind Bleicherden die durch Behandlung mit Salzsäure aktiviert wurden wie beispielsweise die Bleicherde vom Typ Tonsil® K 10 der Firma Südchemie.

Im allgemeinen werden die aktiven Bleicherden durch Behandlung von Bleicherde mit Mineralsäuren erhalten. Von der dabei entstandenen sauren Suspension wird die Säure durch Filtration abgetrennt und der Filterkuchen mit Wasser gewaschen. Die sich daran anschließenden Trocknungs- und Mahlprozesse liefern eine aktive Bleicherde mit einem Restwassergehalt von kleiner 10 %, vorzugsweise kleiner 7 %.

Die aktiven Bleicherden weisen in der Regel Säurezahlen von 10 bis 150 auf, vorzugsweise von 10 bis 80. Die Säurezahl gibt dabei die Anzahl der mg KOH an, die zur Neutralisation von 1 g Bleicherde verbraucht wird. Sie wird in der Regel durch Titration gegen einen Indikator wie Phenolphthalein oder Methylorange oder mit einem pH-Meter ermittelt.

Im allgemeinen beträgt das molare Verhältnis der Anilinverbindung zu Acrylnitril 1,5:1 bis 3,5:1, vorzugsweise 2:1 bis 3:1.

Unter einem nichtwäßrigen Reaktionsmedium wird im allgemeinen ein organisches Reaktionsmedium mit einem Wassergehalt von maximal 0,5 %, vorzugsweise von maximal 0,1 %, verstanden.

In einer bevorzugten Ausführungsform dient die zu Acrylnitril im molaren im Überschuß eingesetzte Anilinverbindung gleichzeitig als nichtwäßriges Reaktionsmedium, wodurch ein zusätzliches Reaktionsmedium nicht erforderlich ist. Das Verfahren wird in der Regel bei einer Temperatur von 80 bis 200 °C, vorzugsweise bei 100 bis 150 °C durchgeführt.

Die Menge an einzusetzendem Katalysator beträgt 1 bis 15 Gew.-%, vorzugsweise 2 bis 6 Gew.-% bezogen auf die Anilinverbindung.

Das Verfahren wird vorzugsweise so durchgeführt, daß die Anilinverbindung zusammen mit dem Katalysator auf die Reaktionstemperatur erhitzt wird und das Acrylnitril langsam zudosiert wird. Die Aufarbeitung des Reaktionsgemisches erfolgt vorzugsweise dadurch, daß der Katalysator bei einer geeigneten Temperatur vorzugsweise durch Filtration aus dem Reaktionsgemisch entfernt wird und die katalysatorfreie Reaktionsmischung fraktioniert destilliert wird. Dabei erhält man die Ausgangsverbindungen Acrylnitril und die Anilinverbindung, die wieder eingesetzt werden können, sowie das Mono-N-$\beta$-cyanoethylanilin, was in einer Reinheit von >99 % anfällt,

Der abfiltrierte Katalysator kann für die Reaktion ebenfalls wiederverwendet werden.

Das erfindungsgemäße Verfahren wird in einem nichtwäßrigen Reaktionsmedium durchgeführt und erzeugt daher keine schädlichen Abwässer. Es ist einfach zu handhaben und liefert reine Produkte in guter Ausbeute.

Die folgenden Beispiele erläutern das erfindungsgemäße Verfahren:

Der in den nachfolgenden Beispielen verwendete Katalysator ist Tonsil® K 10 der Firma Südchemie. Dieser Katalysator ist eine pulverförmige mit Salzsäure aktivierte Bleicherde mit den folgenden physikalischen Angaben:

Oberfläche: 220 bis 270 $m^2/g$

Porenvolumen:

a)

| $CCl_4$ | |
|---|---|
| ml/g<br>nm-Bereich | 0,318<br>0 bis 80 |

b)

| Hg-Porosimeter | | | | |
|---|---|---|---|---|
| ml/g | 0,29 | 0,53 | 0,13 | 0,07 |
| nm-Bereich | 15000-1750 | 1750-80 | 80-14 | 14-7,5 |

Glühverlust: ca. 7 %

Schüttgewicht: 300 bis 370 g/l

Säurezahl: ca. 20

Beispiel 1

N-$\beta$-cyanoethyl-N-methylanilin

In einem 3-Halsrührkolben mit Rückflußkühler und Einleitungsrohr wrden 1071,5 Gew.-Teile (10 Mol) N-Methylanilin und 15,0 Gew.-Teile Tonsil® K 10 vorgelegt. Die Mischung wird auf 120 °C erhitzt und bei dieser Temperatur über ein Einleitungsrohr 265,3 Gew.-Teile (5 Mol) Acrylnitril zugegeben. Die Reaktionsmischung wird 18 Stunden bei 120 bis 130 °C nachgerührt und anschließend auf 60 °C abgekühlt. Der Katalysator wird abfiltriert und die flüssige Phase einer fraktionierten Destillation unterworfen. Es werden 47,7 Gew.-Teile Acrylnitril und 620,9 Gew.-Teile N-Methylanilin wiedergewonnen; sie können als Ausgangsmaterial wieder eingesetzt werden. Die Ausbeute an N-$\beta$-Cyanoethyl-N-methylanilin beträgt 605,0 Gew.-Teile ~ 75.5 % Umsatz. Die Reinheit beträgt 99,5 % N-$\beta$-Cyanoethyl-N-methylanilin.

Beispiel 2

N-β-Cyanoethyl-o-methoxyanilin

Wie in Beispiel 1 beschrieben, werden 307,9 Gew.-Teile o-Methoxyanilin und 12,0 Gew.-Teile Tonsil® K 10 vorgelegt und anschließend bei 140 °C 66,3 Gew.-Teile Acrylnitril zugegeben. Die Mischung wird bei 140 °C 27 Stunden verrührt. Nach der Aufarbeitung beträgt die Ausbeute an N-β-Cyanoethyl-o-methoxyanilin 137,2 Gew.-Teile ≈ 62,3 % Umsatz. Die Reinheit des aufgearbeiteten Produkts beträgt 98,9 % N-β-Cyanoethyl-o-methoxyanilin.

Beispiel 3

N-β-Cyanoethyl-o-ethoxyanilin

Wie in Beispiel 1 beschrieben, werden 342,9 Gew.-Teile o-Ethoxyanilin und 12 Gew.-Teile Tonsil® K 10 vorgelegt und anschließend bei 140 bis 155 °C 66,3 Gew.-Teile Acrylnitril zugegeben. Die Mischung wird danach 27 Stunden bei 170 °C verrührt` Nach der Aufarbeitung beträgt die Ausbeute an N-β-Cyanoethyl-o-ethoxyanilin 125,6 Gew.-Teile ≈ 52,8 % Umsatz mit einer Reinheit von 99,2 %.

Beispiel 4

N-β-Cyanoethyl-o-methylanilin

Wie in Beispiel 1 beschrieben, werden 214,4 Gew.-Teile o-Methylanilin und 9,0 Gew.-Teile Tonsil® K 10 vorgelegt und anschließend bei 140 bis 150 °C 53,0 Gew.-Teile Acrylnitril zugegeben. Die Mischung wird danach 26 Stunden bei 120 °C verrührt. Nach der Aufarbeitung beträgt die Ausbeute an N-β-Cyanoethyl-o-toluidin 58,6 Gew.-Teile ≈ 36,6 % Umsatz mit einer Reinheit von 98,7 %.

**Patentansprüche**

**1.** Verfahren zur Herstellung von Mono-N-β-cyanoethylanilinen, dadurch gekennzeichnet, daß man eine gegebenenfalls substituierte Anilinverbindung mit Acrylnitril in Gegenwart von aktiver Bleicherde als Katalysator bei einer Temperatur zwischen 80 und 200 °C in einem nichtwäßrigen Reaktionsmedium umsetzt.

**2.** Verfahren gemaß Anspruch 1, dadurch gekennzeichnet, daß die Anilinverbindung der Formel (I)

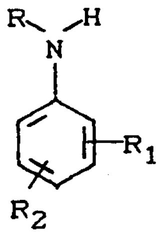

(I)

entspricht, worin

R = Wasserstoff oder gegebenenfalls mit Hydroxy substituiertes $C_1$-$C_{10}$-Alkyl,

$R_1$, $R_2$ = unabhängig voneinander H, $C_1$-$C_{10}$-Alkyl oder $C_1$-$C_{10}$-Alkoxy bedeuten.

**3.** Verfahren gemaß Anspruch 2, dadurch gekennzeichnet, daß Aniline der Formel (I) verwendet werden, worin

R = Wasserstoff, Methyl, Ethyl, Propyl, Isopropyl, β-Hydroxyethyl, β-Hydroxypropyl oder γ-Hydroxypropyl,

$R_1$, $R_2$ = unabhängig voneinander Wasserstoff, Methyl, Ethyl, Isopropyl, Propyl, Methoxy oder Ethoxy bedeutet.

4. Verfahren gemäß Anspruch 2, dadurch gekennzeichnet, daß Aniline der Formel (I) verwendet werden, worin
   R = Wasserstoff oder Methyl,
   $R_1$ = Wasserstoff, Methyl, Methoxy oder Ethoxy und
   $R_2$ = Wasserstoff bedeuten.

5. Verfahren gemäß Anspruch 1, dadurch gekennzeichnet, daß man als Katalysator Bleicherden verwendet, die durch Behandlung mit Salzsäure aktiviert wurden.

6. Verfahren gemaß Anspruch 1, dadurch gekennzeichnet, daß man 1 bis 15 Gew.-% Katalysator bezogen auf die Anilinverbindung einsetzt.

7. Verfahren gemäß Anspruch 1, dadurch gekennzeichnet, daß das molare Verhältnis der Anilinverbindung zu Acrylnitril 1,5:1 bis 3,5:1 beträgt.

8. Verfahren gemäß Anspruch 1, dadurch gekennzeichnet, daß als nichtwäßriges Reaktionsmedium die zu Acrylnitril im molaren Überschuß eingesetzte Anilinverbindung dient.

Europäisches Patentamt

# EUROPÄISCHER RECHERCHENBERICHT

Nummer der Anmeldung
EP 94 10 0940

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| X | US-A-3 943 162 (BRENNAN ET. AL.)<br>* Spalte 2, Zeile 63 - Spalte 3, Zeile L; Ansprüche; Beispiele *<br>--- | 1-8 | C07C253/30<br>C07C255/24 |
| A | DE-A-19 47 933 (EASTMAN KODAK CO.)<br>* Ansprüche; Beispiele *<br>--- | 1-8 | |
| D,A | US-A-3 496 213 (ROSS)<br>* das ganze Dokument *<br>----- | 1-8 | |
| | | | RECHERCHIERTE SACHGEBIETE (Int.Cl.5) |
| | | | C07C |

Der vorliegende Recherchenbericht wurde für alle Patentansprüche erstellt

| Recherchenort | Abschlußdatum der Recherche | Prüfer |
|---|---|---|
| DEN HAAG | 6. Mai 1994 | Helps, I |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)